# EUROPEAN PATENT SPECIFICATION

(11) **EP 2 067 466 B1**
(45) Date of publication and mention of the grant of the patent: **15.08.2012**
(21) Application number: 07122104.8
(22) Date of filing: 03.12.2007
(51) Int. Cl.: A61K 8/04, A61K 8/73, A61K 8/891, A61Q 19/00, A61Q 19/10, A61K 9/14

(54) **Cosmetic and/or dermatological powder comprising modified starch**
Kosmetisches und/oder dermatologisches Pulver enthaltend modifizierte Stärke
Poudre cosmétique et/ou dermatologique comprenant un amidon modifié

(43) Date of publication of application: 10.06.2009
(73) Proprietor: Vongsurakrai, Varatus, Bangkok 10700 (TW)
(72) Inventor: Vongsurakrai, Varatus, 10700, Bangkok (TH); Varavinit, Saiyavit, 10400, Bangkok (TH)
(74) Representative: TBK

(56) References cited:
- WO-A1-98/19652
- US-A- 2 876 160
- US-A- 4 568 539
- US-A- 5 720 978
- US-A- 5 776 476
- US-A- 5 783 211
- US-A1- 2002 110 875
- US-A1- 2005 070 502
- US-A1- 2006 280 714

## Description

### BACKGROUND OF THE INVENTION

### 1. Field of the Invention

The present invention relates to a cosmetic or dermatological powder containing at least one Aluminium Starch Octenyl Succinate, to a process for its manufacture and to a composition containing it. More particularly, the invention relates to the use of the powder or of the composition for the care and/or treatment of the skin, mucous membranes and/or the scalp, as well as for the treatment of skin disorders.

### 2. Description of the Background

Compositions containing Aluminium Starch Octenyl Succinate, which are useful for their beneficial effects on the skin, are known in cosmetics and dermatology. Certain modified starch makes it possible, in particular, to treat problems of sensitive skin. Moreover, it is known that the incorporation of Aluminium Starch Octenyl Succinate into cosmetic or dermatological compositions providing excellent water repellant, fat & oil absorbency, skin asperity and friction reduction properties, which is suitable for human skin.

US-A-5 720 978 is directed to encapsulated products comprising a matrix of a starch hydrolyzate acid ester having a water-insoluble material encapsulated therein.

US-A-5 776 476 is directed to cosmetic compositions useful in skin care wherein in one Example of this document, a translucent face powder containing a dodecenyl starch derivative is formulated.

US-A-2005/070 502 discloses compositions for the treatment of dysglucaemia. According to one Example of this document in the method of producing the composition, a native cornstarch is added to water, dried and up to 10% olive oil is added to the granulated product.

US-A-2 876 160 describes a starch matrix material containing an embedded material as well as the process for producing the same wherein in the examples of this document modified starch is mixed with oily components.

US-A-2002/110 875 discloses a fluid absorber and a method for preparing the same, which is used for cosmetic purposes and wherein a modified starch is used to absorb a oleogenous flavouring agent.

US-A-4 568 539 discloses a body powder composition consisting of 99 to 80% starch and 1 to 20% of a pregelatinized cornstarch and which may also contain a perfume in an amount from about 0.01 to 1.0% by weight.

US 2006280714 (A1) describes a small-granule aluminum starch octenylsuccinate (ASO) derivative having a distribution of starch granules with a mean diameter of between 4.5 mum and 8.9 mum is disclosed herein. The small-granule ASO may be used in a wide variety of personal care formulations.

US 5783211 (A) describes a dry powder preferably prepared by a process comprising spray-drying a mixture of liposome encapsulated active agent, starch and maltodextrin. The particle is designed so that activity of, e.g., an antiinflammatory agent such as Dragosantol TM can be specifically triggered by skin conditions, such as moisture, for optimal timing of delivery.

WO 9819652 (A1) relates to a spray-dried powder compatible with an anhydrous or hydrophobic non-oral composition comprises a substantially water-soluble absorbent matrix containing a protein and a hydrolysed starch.

### SUMMARY OF THE INVENTION

Briefly, this object and other objects of the present invention as hereinafter will become more readily apparent can be attained by a cosmetic or dermatological powder, which is defined according to claim 1.

### DETAILED DESCRIPTION OF THE PREFERRED EMBODIMENTS

The term "powder" in this invention means a solid substance divided into very fine, homogeneous particles or grains. The powder of the invention is preferably low protein flour which contain at the maximum of 10.0% protein.

In one specific embodiment of the invention the powder comprises from 61 to 99.99% by weight relative to the total weight of the powder of at least one modified starch, and from 0.01 to 29% by weight relative to the total weight of the powder of an oily phase comprising at least one oil and a preservative.

The cosmetic or dermatological powder of the invention comprises an oily phase fixed in the modified starch. This powder in particular has the advantages of water repellant, fat & oil absorbency, skin asperity and friction reduction properties, which is suitable for human skin. Moreover, the powder of the invention is not sensitive to moisture or to bacteriological contamination. In addition, since this powder can be obtained without any emulsifier and since it keeps well, it is possible to avoid the addition of emulsifiers and/or preserving agents, and thus to obtain a powder which is much less of an irritant than the conventional skin care products.

In addition, the cosmetic product of the invention allows the incorporation of compounds therein which have different physicochemical properties. This product can thus comprise detergents. This makes it possible, in particular, to cleanse the skin while at the same time moisturizing and nourishing the skin. In fact, the present invention represents a significant contribution to the art since it provides a multipurpose cosmetic or dermatological product which is not specific to one skin type.

The powder of the invention has a particle size or number-average particle size which can range in particular from about 0.1 to 100 micron, preferably from 0.5 to 50 micron, preferably from 1 to 10 micron, this particle size being measured using a Malvern Laser Particle Size Analyzer.

The modified starch employed in the powder of the invention is modified by a combination of esterification and heat-moisture treatment. these reactions are conducted in the following manner:

Esterification in an alkaline medium using octenyl succinic anhydride in order to graft functional groups, in particular octenylsuccinic, onto the starch and follow by the addition of aluminium sulfate to form Aluminium Starch Octenyl Succinate. The modified starch suspension is washed with water by the hydrocyclones or separators in order to eliminate the excess octenyl succinic anhydride and aluminium sulfate, dewatered by filter press, centrifuge, or plain vacuum belt press and dried by paddle drier at lower temperature (not over 80°C) to 27% moisture content. The modified starch is then heated to the temperature not lower than 121°C, which is called "Heat / Moisture -Treatment" in parallel with sterilization, to dryness.

Suitable chemical modified starches which are used in the invention are starches esterified with octenylsuccinic anhydride, namely *"**Aluminium Starch octenyl succinate"*** and followed by a physical modification namely "Heat / Moisture -Treatment" (HMT). HMT process provides completely of the water repellant property of the aluminium starch octenyl succinate.

### EXAMPLE

### Example 1

Skin Care Powder

Sterilized modify-starch 99.99% and Silicone oil 0.01 %

### Procedure

A mixture containing 99.99% of sterilized modify-starch and 0.01 % of the silicone oil with fragrance is incorporated to the starch by spraying.

The powder can be used in its existing, non-reconstituted form, and constitutes a product which is effective for sensitive skin, irritated skin and greasy skin. Furthermore, the powder has the advantage of providing excellent water repellant, fat & oil absorbency, skin asperity and friction reduction properties, which is suitable for human skin.

## Claims

1. A cosmetic or dermatological powder, which comprises:
(i) from 61 to 99.99% by weight relative to the total weight of the powder of at least one starch modified by heat/moisture-treatment, pregelatinization, oxidation, crosslinking, esterification or a combination thereof, (ii) from 0.01 to 29.99% by weight relative to the total weight of the powder of an oily phase comprising at least one oil **characterized in that** the modified starch is the aluminium salt of the reaction product of 1-octenylsuccinic anhydride and starch, wherein the modified starch is obtainable by esterification of starch in an alkaline medium using octenyl succinic anhydride in order to graft functional groups, in particular octenylsuccinic, onto the starch, followed by addition of aluminium sulfate to form aluminium starch octenyl succinate, the modified starch suspension is washed with water by hydrocyclones or separators in order to eliminate the excess octenyl succinic anhydride and aluminium sulfate, dewatered by filter press, centrifuge or plain vacuum belt press and dried by paddle drier at lower temperature not over 80°C to 27% moisture content, and the modified starch is then heated to the temperature not lower than 121°C to dryness.

2. The powder according to claim 1, which is low protein flour, consisting of 0.01 to 10.00% protein.

3. The powder according to claim 1, wherein the oily phase comprises at least one oil selected from the group consisting of mineral oils, silicone oils, oils of plant origin, and oils of animal origin and synthetic oils.

4. The powder according to claim 1 consisting of not more than 10.00%preservative such as zinc oxide.

## Patentansprüche

1. Kosmetisches oder dermatologisches Pulver, welches umfasst:
(i) von 61 bis 99,99 Gew.-%, relativ zu dem Gesamtgewicht des Pulvers, von zumindest einer Stärke, die durch Wärme-/Feuchtigkeits-Behandlung Vorgelatisierung, Oxidation, Quervernetzung, Veresterung oder einer Kombination davon, modifiziert ist, (ii) von 0,01 bis 29,99 Gew.-%, relativ zu dem Gesamtgewicht des Pulvers, von einer öligen Phase, die zumindest ein Öl umfasst, **dadurch gekennzeichnet, dass** die modifizierte Stärke das Aluminiumsalz des Reaktionsprodukts von 1-Octenylbernsteinsäureanhydrid und Stärke ist, wobei die modifizierte Stärke erhältlich ist durch Veresterung von Stärke in einem alkalischen Medium unter Verwendung von Octenylbernsteinsäureanhydrid, um funktionelle Gruppen aufzupfropfen, insbesondere Octenylbernsteinsäure, auf die Stärke, gefolgt durch die Zugabe von Aluminiumsulfat, um Aluminiumstärkeoctenylsuccinat zu bilden, wobei die modifizierte Stärkesuspension mit Wasser durch Hydrozyklone oder Abscheider gewaschen wird, um das überschüssige Octenylbernsteinsäureanhydrid und Aluminiumsulfat zu eliminieren, durch eine Filterpresse, Zentrifuge oder eine Vakuumflachgürtelpresse entwässert wird und durch einen Schaufeltrockner bei niedriger Temperatur nicht über 80°C auf 27% Feuchtigkeitsgehalt getrocknet wird, und die modifizierte Stärke dann zu einer Temperatur von nicht weniger als 121°C bis zur Trockenheit erwärmt wird.

2. Pulver nach Anspruch 1, welches ein Mehl mit niedrigem Proteingehalt ist, das aus 0,01 bis 10,00% Protein besteht.

3. Pulver nach Anspruch 1, wobei die ölige Phase zumindest ein Öl umfasst, das aus der Gruppe ausgewählt ist, die aus Mineralölen, Silikonölen, Ölen pflanzlichen Ursprungs und Ölen tierischen Ursprungs und synthetischen Ölen besteht.

4. Pulver nach Anspruch 1, das aus nicht mehr als 10,00% Konservierungsstoffen, wie etwa Zinkoxid, besteht.

## Revendications

1. Poudre dermatologique ou cosmétique, qui comprend :
(i) de 61 à 99,99% en poids par rapport au poids total de la poudre d'au moins un amidon modifié par traitement hydro-thermique, pré-gélatinisation, oxydation, réticulation, estérification ou une combinaison de ceux-ci, (ii) de 0,01 à 29,99% en poids par rapport au poids total de la poudre d'une phase huileuse comprenant au moins une huile **caractérisée en ce que** l'amidon modifié est le sel d'aluminium du produit réactionnel de l'anhydride 1-octénylsuccinique et de l'amidon, où l'amidon modifié peut être obtenu par estérification de l'amidon dans un milieu alcalin en utilisant l'anhydride octényl-succinique afin de greffer des groupes fonctionnels, en particulier le groupe octényl-succinique, sur l'amidon, suivi par l'addition du sulfate d'aluminium pour former l'octényl succinate d'amidon d'aluminium, la suspension d'amidon modifié est lavée avec l'eau par des hydrocyclones ou des séparateurs, afin d'éliminer l'anhydride octényl-succinique et le sulfate d'aluminium en excès, déshydratée par filtre-presse, presse à bande sous vide lisse ou centrifuge et séchée par un séchoir à palettes à une basse température qui ne dépasse pas 80°C à une teneur en humidité de 27%, et l'amidon modifié est ensuite chauffé à une température non inférieure à 121°C jusqu'à siccité.

2. Poudre selon la revendication 1, qui est une farine à faible teneur en protéine, composée de 0,01 à 10,00% de protéines.

3. Poudre selon la revendication 1, dans laquelle la phase huileuse comprend au moins une huile choisie dans le groupe composé d'huiles minérales, d'huiles de silicone, d'huiles d'origine végétale, d'huiles d'origine animale et d'huiles de synthèse.

4. Poudre selon la revendication 1 ne contenant pas plus de 10,00% de conservateur tel que l'oxyde de zinc.
